# FASCICULE DE BREVET EUROPEEN

(11) **EP 3 658 085 B1**
(45) Date de publication et mention de la délivrance du brevet: **28.06.2023**
(21) Numéro de dépôt: 18755528.9
(22) Date de dépôt: 27.07.2018
(51) Int. Cl.: A61F 5/455

(54) **COUPE MENSTRUELLE RAINUREE, PROCEDE DE FABRICATION, KIT ET METHODE DE DESINFECTION ASSOCIES**
GERILLTER MENSTRUATIONSBECHER, HERSTELLUNGSVERFAHREN DAFÜR UND ZUGEHÖRIGES DESINFEKTIONSVERFAHREN UND KIT
GROOVED MENSTRUAL CUP, PRODUCTION METHOD THEREOF, AND ASSOCIATED DISINFECTION METHOD AND KIT

(30) Priorité: 28.07.2017 FR 1770805
(43) Date de publication de la demande: 03.06.2020
(73) Titulaire: Claripharm, 22400 Saint Alban (FR)
(72) Inventeur: LE COURT, Clarisse, 22370 Pleneuf-Val-Andre (FR)
(74) Mandataire: Rhein, Alain
(86) Numéro de dépôt international: PCT/FR2018/051934
(87) Numéro de publication internationale: WO 2019/020958

(56) Documents cités:
- FR-A1- 2 187 280
- GB-A- 2 425 260
- US-A- 5 295 984
- US-A- 5 947 992

## Description

La présente invention concerne une coupe menstruelle et un procédé de fabrication associé. L'invention concerne en outre une méthode de désinfection de la coupe menstruelle.

Le cycle menstruel de femmes post-pubertes implique des écoulements de menstrues de manière périodique, générant des inconvénients de confort et de praticité.

Une première solution connue est d'utiliser des serviettes hygiéniques ou des tampons. Une autre solution alternative a été proposée, et est basée sur l'utilisation de coupes menstruelles, à savoir des dispositifs en forme de récipients insérés dans la cavité vaginale afin de recueillir des écoulements de menstrues.

Malheureusement, certaines utilisatrices peuvent avoir des règles dites abondantes, c'est-à-dire un flux d'écoulement supérieur à la normale statistique. Ces règles abondantes peuvent dépasser la capacité de contenance de la coupe menstruelle lors de la durée maximale d'utilisation et se déverser en dehors de cette dernière.

Les documents US 5,947,992, US 5,295,984, FR 2 187 280 et GB 2 425 260 décrivent des coupes menstruelles connues de l'état de l'art.

Ainsi, un premier objectif de la présente invention est de proposer une coupe menstruelle dont la capacité de contenance est augmentée sans augmenter significativement le diamètre maximal de la coupe menstruelle.

En outre, pour des utilisatrices ayant des déficiences de tonicité du périnée ou de la paroi vaginale ou leurs affaiblissements, le maintien en place de la coupe menstruelle peut être compromis, ce qui peut conduire là aussi à des déplacements de la coupe menstruelle et des écoulements indésirés. En effet, plus le périnée est affaibli, moins il est facile de maintenir la coupe menstruelle en place dans la cavité vaginale.

Ainsi, un deuxième objectif de la présente invention est de proposer une coupe menstruelle permettant de contrecarrer des déficiences de tonicité du périnée ou de la paroi vaginale ou leurs affaiblissements.

Afin de pallier aux inconvénients de l'art antérieur, l'invention propose une coupe menstruelle comprenant une ouverture supérieure, et un réceptacle comprenant un corps en forme longitudinale arrondie et un fond, la coupe s'étendant longitudinalement de l'ouverture au fond et présentant une face interne dans la concavité du réceptacle, et une face externe dans la convexité du réceptacle.

Selon un premier aspect, le corps comprend au moins une rainure longitudinale creuse sur la face interne du corps.

Avantageusement, la rainure longitudinale creuse permet d' augmenter la capacité de contenance de la coupe menstruelle sans pour autant augmenter la taille de la coupe menstruelle.

Selon d'autres aspects pris isolément ou combinés selon toutes les combinaisons techniquement réalisables :
- la coupe comprend une pluralité de rainures longitudinales creuses et/ou pleines ; et/ou
- lesdites rainures creuses avec lesquelles coïncident des rainures pleines, sont réparties de manière régulière en périphérie du corps ; et/ou
- la coupe comporte quatre rainures creuses avec lesquelles correspondent quatre rainures pleines ; et/ou
- la coups comporte au moins une rainure présentant une section arrondie ou angulaire ; et/ou
- la coupe comporte au moins une rainure présentant une forme générale évasée, en forme de poire ou ovoïde ; et/ou
- la coupe comporte au moins une rainure présentant une forme générale de serpentin ; et/ou
- au moins une rainure présente une forme tubulaire de section correspondant a environ un tiers de celle de l'ouverture ; et/ou
- la coupe comprend une section évasée vers la haut entre le corps et l'ouverture ; et/ou
- ladite section évasée présente une augmentation de diamètre d'au moins environ 25% entre le diamètre maximal du corps et le diamètre extérieur de la section évasée.

L'invention porte en outre sur un kit comprenant une coupe menstruelle selon l'invention configurée pour être résistante aux microondes, et un récipient de stockage résistant aux microondes.

Un autre objet de l'invention concerne un procédé de fabrication d'une coupe menstruelle selon l'invention, comprenant une étape de formation d'au moins une rainure longitudinale pleine et d'au moins une rainure longitudinale creuse, caractérisée par une mise en forme desdites rainures au moyen d'un moule comprenant une rainure longitudinale pleine correspondante.

L'invention concerne en outre une méthode de désinfection d'une coupe menstruelle selon l'invention, configurée pour être résistante aux microondes, la méthode comprenant de préférence au moins une étape de nettoyage de la coupe menstruelle ; une étape de mise en place de la coupe menstruelle dans un récipient de stockage résistant aux microondes ; et une étape d'application de microondes sur la coupe et le récipient de sorte à désinfecter la coupe menstruelle.

L'invention sera davantage détaillée par la description de modes de réalisation non limitatifs, et sur la base des figures annexées, dans lesquelles :
- la figure 1 est une vue dans l'espace d'une coupe menstruelle selon une première variante de l' invention ;
- la figure 2 est une vue en plan de la coups menstruelle de la figure 1 ;
- la figure 3 est une vue en coupe selon AA de la coupe menstruelle de la figure 2 ;
- la figure 4 est une vue de dessous de la coupe menstruelle des figures 1 à 3 ;
- la figure 5 est une vue dans l'espace d'une coupe menstruelle selon une deuxième variante de l' invention ;
- la figure 6 est une vue en plan de la coupe menstruelle de la figure 5 ;
- la figure 7 est une vue en coupe selon AA de la coupe menstruelle de la figure 6 ;
- la figure 8 est une vue de dessous de la coupe menstruelle des figures 5 à 7 ;
- la figure 9 est une vue dans l'espace illustrant la méthode de désinfection selon l'invention.

La présente invention concerne une coupe menstruelle 1 améliorée par rapport à l'art antérieur.

La coupe menstruelle 1 comprend une ouverture supérieure 2, et un réceptacle 3. L'ouverture supérieure 2 est délimitée par une collerette 2a. Le réceptacle 3 a une forme de récipient et est configuré pour contenir les écoulements menstruels arrivant par l'ouverture 2. La section de l'ouverture 2 et/ou celle du réceptacle 3 peut être de forme quelconque, par exemple en forme polygonale telle qu'une forme carrée, rectangulaire, triangulaire, trapézoïde, ou en forme arrondie, telle qu'une forme de cercle ou une forme ovoïde. La forme circulaire est davantage préférée pour plus de confort dans la paroi vaginale.

Le réceptacle 3 comprend un corps 4 ayant une forme longitudinale arrondie et un fond 5 correspondant en particulier au fond du récipient. La corps 4 est en particulier la partie configurée pour épouser la paroi vaginale. Le fond 5 est la partie où tombe les premiers écoulements menstruels, et la coupe 1 se remplit du fond 5 vers l'ouverture 2 au fur et à mesure desdits écoulements.

Le réceptacle peut comprendre un plan de symétrie ou de préférence un axe de symétrie, en particulier un axe de symétrie de rotation. Cet axe est au centre du réceptacle 3.

La coupe menstruelle 1 s'étend longitudinalement de l'ouverture 2 vers le fond 5. La coupe 1 peut également s'étendre latéralement, mais l'axe longitudinal est considéré comme celui allant de l'ouverture 2 vers le fond 5 de la coupe menstruelle 1.

La coupe menstruelle 1 présente une face interne 6 dans la concavité du réceptacle 3, et une face externe 7 dans la convexité du réceptacle 3.

Selon l'invention, le corps 4 comprend une pluralité de rainures longitudinales creuses 8, sur la face interne 6 du corps 4.

Avantageusement, la pluralité de rainures longitudinales creuses 8 permet d'augmenter la capacité de contenance de la coupe menstruelle 1 sans pour autant augmenter le diamètre maximal de la coupe menstruelle 1.

Selon une variante, la coupe 1 comprend en outre une pluralité de rainures longitudinales pleines 9 sur la face externe 7 du corps 4.

Avantageusement, la pluralité de rainures longitudinales pleines 9 permet de contrecarrer des déficiences de tonicité du périnée ou de la paroi vaginale ou leurs affaiblissements. La coupe menstruelle 1 tient ainsi mieux en place dans la cavité vaginale notamment en raison de l'augmentation de la surface de contact entre la coupe 1 et la paroi vaginale en raison de ladite rainure pleine.

Selon une variante, ladite pluralité de rainures longitudinales pleines 9 sur la face externe 7 coïncide avec ladite pluralité de rainures longitudinales creuses 8 sur la face interne 6. Avantageusement, cet aspect permet de réaliser les rainures creuses 8 et les rainures pleines 9 de taille significative et ainsi améliorer l'efficacité sur la contenance et le maintien dans la paroi vaginale.

Selon une variante, la coupe 1 comporte quatre rainures longitudinales creuses 8 avec lesquelles correspondent quatre rainures pleines 9. Plus particulièrement ces rainures sont réparties régulièrement sur la périphérie du corps 4.

Une répartition régulière des rainures 8 ; 9 permet de bénéficier de l'avantage de ladite rainure creuses 8 et/ou pleines 9 à plusieurs endroits de la coupe menstruelle 1.

Prévoir quatre rainures permet d'avoir un bon compromis entre le nombre de ces dernières et leur section.

Selon une variante, au moins une rainure présente une forme d'élément tubulaire ayant une épaisseur correspondant à environ un tiers de celle de l'ouverture. La rainure mesure par exemple environ dix millimètres. Avantageusement, les rainures ont une taille significative ce qui permet de bénéficier pleinement de l'effet sur la contenance et/ou le maintien, en particulier sur les parois ayant un défaut de tonicité ou un d'affaiblissement.

Plus généralement, au moins une rainure 8, 9 peut présenter une section arrondie ou angulaire.

La forme générale de la rainure peut être évasée, en forme de poire, ou ovoïde. Par exemple, les rainures peuvent avoir une taille fine vers l'ouverture et une taille plus large vers la fond pour améliorer davantage la tenue de la coupe menstruelle dans la cavité vaginale. Une section arrondie peut être associée à une forme générale n'étant pas circulaire. Par exemple, une forme de serpentin peut être envisagée.

En particulier, les quatre rainures creuses et pleines ont de préférence une même forme.

Selon une variante, la coupe 1 comprend une section évasée 9a vers le haut entre le corps 4 et l'ouverture 2, en particulier la collerette 2a. La section évasée 9a comprend en particulier une forme de tulipe. La section évasée 9a permet d'augmenter davantage la contenance de la coupe menstruelle 1 et d'avoir également un meilleur maintien dans la cavité vaginale en raison de la forme évasée s'opposant au retrait de la coupe menstruelle 1.

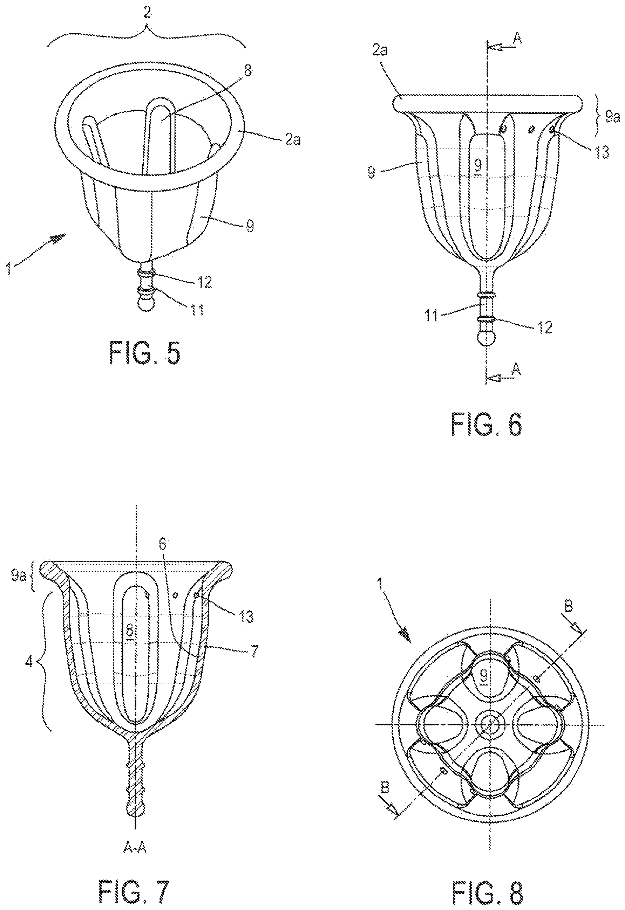

Selon une variante, la section évasée 9a présente une augmentation de diamètre de plus d'environ 25% entre le diamètre maximal du corps et le diamètre extérieur de la section évasée, de préférence de plus de 50% et plus préférentiellement moins de 80%. Dans la variante illustrée aux figures 5 à 8, une augmentation de diamètre de 66%, de 30 mm à 50 mm, a été réalisée et donne de très bons résultats.

La forme évasée a été étudiée pour s'adapter parfaitement à l'anatomie vaginale et la collerette supérieure en forme d'anneau est renforcée pour optimiser l'ouverture de la coupe 1 et le maintien ouvert permettant l'étanchéité avec la paroi vaginale.

Afin d'avoir une protection fiable, le choix de la coupe menstruelle est fonction de la tonicité du périnée. De là, deux critères sont à prendre en considération : le diamètre de la coupe et sa flexibilité relative. En effet, cette coupe peut être définie plus ou moins souple, voire même rigide.

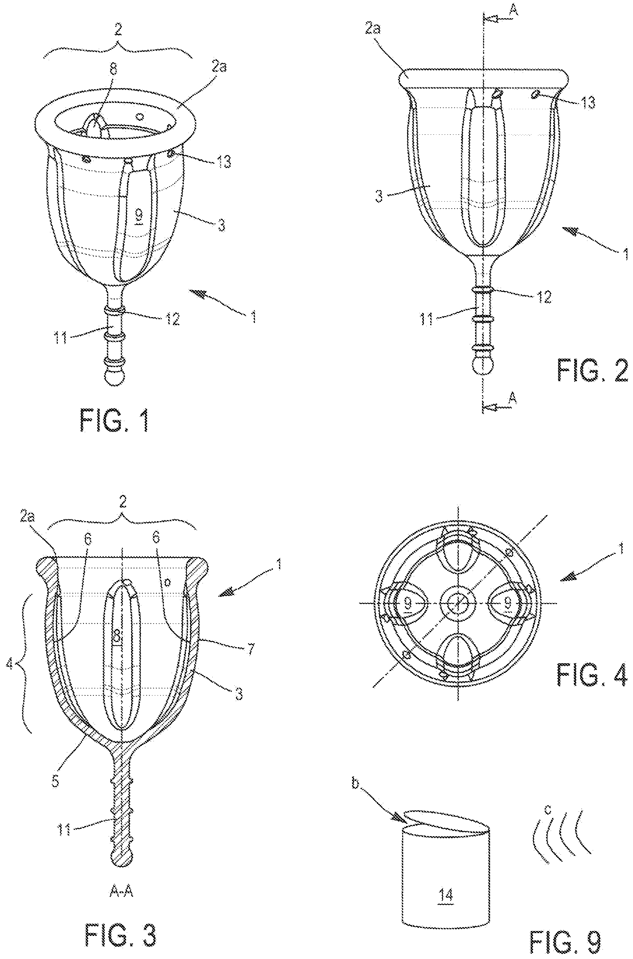

Plusieurs tailles de diamètres de coupe menstruelle 1 peuvent être envisagées afin de pouvoir s'adapter à la majorité des femmes selon leur anatomie vaginale. Par exemple, le diamètre externe peut être de 41mm, 46mm, 51mm, ou 56mm. La coupe 1 de la figure 1 a un diamètre externe de 41 mm et un diamètre interne de 31mm. Celle de la figure 5 a un diamètre de 56mm au niveau de l'ouverture 2 et un diamètre interne de 31mm au niveau du corps 4.

La coupe menstruelle 1 peut comprendre une tige 11 solidaire du fond 5. La tige 11 comprend de préférence des reliefs 12 à intervalles réguliers. La tige 11 est ajustée à la sortie de la cavité vaginale lorsque la coupe menstruelle 1 y est mise en place. Une partie s'étendant à l'extérieur du vagin peut être découpée et la partie restante sert à retirer la coupe menstruelle.

La coupe menstruelle 1 peut comprendre des orifices 13 en dessous de l'ouverture 2, en particulier de la collerette 2a, permettant de diminuer les effets de ventouse entre la coupe et la paroi vaginale.

De préférence, la coupe menstruelle 1 est réalisée en matériau thermoplastique non-irritant pour la paroi vaginale. Plus préférentiellement, le matériau de la coupe menstruelle est configuré pour permettre de plier la coupe 1, la disposer dans la cavité vaginale et la laisser reprendre sa forme initiale. Par exemple, le matériau comprend de la silicone médicale. Au moins un polymère usuel peut être utilisé, ou un mélange de polymères. Les polymères des familles des TPE thermoplastiques élastomères et des TPU polyuréthane thermoplastiques peuvent être sélectionnés. Le matériau qu'est la silicone à usage médical est préféré pour avoir plus de confort en utilisation.

Une fois mis en place dans la cavité vaginale, le réceptacle entre en contact avec la paroi vaginale et les écoulements menstruels entrent par l'ouverture et se déposent dans le réceptacle.

La coupe menstruelle 1 préférée mesure environ 5 cm de long et de 3 à 5 cm de large en fonction de la présence ou non de la forme évasée.

La capacité de la coupe menstruelle préférée est d'environ 15 à 50 ml en fonction de la présence ou non de la forme évasée.

La coupe menstruelle 1 selon l'invention peut être maintenue dans la cavité vaginale en dépit de règles abondantes pendant une durée maximale d'utilisation généralement fixée à environ à 12 heures pour éviter le syndrome de choc toxique. Une coupe menstruelle connue sans rainure ni forme évasée présente une contenance significativement moindre.

Après utilisation, la coupe menstruelle 1 peut être lavée, mise dans une boite de rangement (b) et désinfectée (c) dans un four à micro-ondes.

Ainsi, l'invention porte en outre sur un kit comprenant une coupe menstruelle 1 telle que décrite ci-dessus configurée pour être résistante aux microondes, et un récipient de stockage 13 résistant aux microondes. La méthode de désinfection associée fait également partie de l'invention.

L'invention porte en outre sur un procédé de fabrication d'une coupe menstruelle 1 telle que décrite ci-dessus, comprenant une étape de formation d'au moins une rainure longitudinale pleine et d'au moins une rainure longitudinale creuse, caractérisée par une mise en forme desdites rainures au moyen d'un moule comprenant une rainure longitudinale pleine correspondante. Dans une variante, la mise en forme comprend en particulier une polymérisation du matériau de la coupe menstruelle. Plus particulièrement, un moule mâle et un moule femelle sont utilisés, et la polymérisation est effectuée entre les deux moules.

Selon une autre variante envisageable, la coupe menstruelle peut être réalisée par impression 3D au moyen d'une imprimante 3D connue en elle-même de l'homme du métier.

## Revendications

1. Coupe menstruelle (1) comprenant une ouverture supérieure (2), et un réceptacle (3) comprenant un corps (4) de forme longitudinale arrondie et un fond (5), la coupe s'étendant longitudinalement de l'ouverture (2) au fond (5) et présentant une face interne (6) dans la concavité du réceptacle (3), et une face externe (7) dans la convexité du réceptacle (3),
le corps (4) comprenant au moins une rainure longitudinale creuse (8) sur la face interne (6) du corps (4), **caractérisée en ce que** le corps comprend au moins une rainure longitudinale pleine (9) sur la face externe (7) du corps (4) qui coïncide avec ladite rainure longitudinale creuse (8) sur la face interne (6).

2. Coupe menstruelle (1) selon la revendication 1, **caractérisée en ce qu'**elle comprend une pluralité de rainures longitudinales creuses (8) et/ou pleines (9).

3. Coupe menstruelle (1) selon la revendication 2, **caractérisée en ce que** les rainures creuses (8) avec lesquelles coïncident des rainures pleines (9), sont réparties de manière régulière en périphérie du corps (4).

4. Coupe menstruelle (1), selon l'une quelconque des revendications précédentes, **caractérisée en ce qu'**elle comporte quatre rainures creuses (8) avec lesquelles correspondent quatre rainures pleines (9).

5. Coupe menstruelle (1) selon l'une quelconque des revendications précédentes, **caractérisée en ce qu'**elle comporte au moins une rainure (8, 9) présentant une section arrondie ou angulaire.

6. Coupe menstruelle (1) selon l'une quelconque des revendications précédentes, **caractérisée en ce qu'**elle comporte au moins une rainure (8,9) présentant une forme générale évasée, en forme de poire ou ovoïde.

7. Coupe menstruelle (1) selon la revendication 1 ou 2, **caractérisée en ce qu'**elle comporte au moins une rainure (8, 9) présentant une forme générale de serpentin.

8. Coupe menstruelle (1) selon la revendication 1 ou 2, **caractérisée en ce qu'**au moins une rainure (8) présente une forme tubulaire de section correspondant à environ un tiers de celle de l'ouverture.

9. Coupe menstruelle (1) selon l'une quelconque des revendications précédentes, **caractérisée en ce qu'**elle comprend une section évasée (9a) vers le haut entre le corps (4) et l'ouverture (2).

10. Coupe menstruelle (1) selon la revendication précédente, **caractérisée en ce que** la section évasée (9a) présente une augmentation de diamètre d'au moins environ 25% entre le diamètre maximal du corps et le diamètre extérieur de la section évasée (9a) .

11. Kit comprenant une coupe menstruelle (1) selon l'une quelconque des revendications précédentes configurée pour être résistante aux microondes, et un récipient de stockage (14) résistant aux microondes.

## Patentansprüche

1. Menstruationstasse (1), umfassend eine obere Öffnung (2) und einen Behälter (3), umfassend einen Körper (4) mit einer abgerundeten Längsform und einen Boden (5), wobei sich die Tasse in Längsrichtung von der Öffnung (2) zu dem Boden (5) erstreckt und eine Innenseite (6) in der Konkavität des Behälters (3) und eine Außenseite (7) in der Konvexität des Behälters (3) aufweist,
der Körper (4) umfassend mindestens eine hohle Längsrille (8) auf der Innenseite (6) des Körpers (4), **dadurch gekennzeichnet, dass** der Körper mindestens eine massive Längsrille (9) auf der Außenseite (7) des Körpers (4) umfasst, die mit der hohlen Längsrille (8) auf der Innenseite (6) übereinstimmt.

2. Menstruationstasse (1) nach Anspruch 1,
**dadurch gekennzeichnet, dass** sie eine Vielzahl von hohlen (8) und/oder massiven (9) Längsrillen umfasst.

3. Menstruationstasse (1) nach Anspruch 2,
**dadurch gekennzeichnet, dass** die hohlen Rillen (8), mit denen die massiven Rillen (9) übereinstimmen, gleichmäßig an einem Umfang des Körpers (4) verteilt sind.

4. Menstruationstasse (1), nach einem der vorstehenden Ansprüche,
**dadurch gekennzeichnet, dass** sie vier hohle Rillen (8) vorweist, denen vier massive Rillen (9) entsprechen.

5. Menstruationstasse (1), nach einem der vorstehenden Ansprüche,
**dadurch gekennzeichnet, dass** sie mindestens eine Rille (8, 9) vorweist, die einen abgerundeten oder winkelförmigen Abschnitt aufweist.

6. Menstruationstasse (1) nach einem der vorstehenden Ansprüche,
**dadurch gekennzeichnet, dass** sie mindestens eine Rille (8, 9) vorweist, die eine allgemeine konisch erweiterte, birnen- oder eiförmige Form aufweist.

7. Menstruationstasse (1) nach Anspruch 1 oder 2,
**dadurch gekennzeichnet, dass** sie mindestens eine Rille (8, 9) vorweist, die eine allgemeine Schlangenform aufweist.

8. Menstruationstasse (1) nach Anspruch 1 oder 2,
**dadurch gekennzeichnet, dass** mindestens eine Rille (8) eine Röhrenform mit einem Querschnitt aufweist, der etwa einem Drittel des der Öffnung entspricht.

9. Menstruationstasse (1) nach einem der vorstehenden Ansprüche,
**dadurch gekennzeichnet, dass** sie einen nach oben konisch erweiterten Abschnitt (9a) zwischen dem Körper (4) und der Öffnung (2) umfasst.

10. Menstruationstasse (1) nach dem vorstehenden Anspruch,
**dadurch gekennzeichnet, dass** der konisch erweiterte Abschnitt (9a) eine Durchmesserzunahme von mindestens etwa 25 % zwischen dem maximalen Durchmesser des Körpers und dem Außendurchmesser des konisch erweiterten Abschnitts (9a) aufweist.

11. Kit, umfassend eine Menstruationstasse (1) nach einem der vorstehenden Ansprüche, die konfiguriert ist, um mikrowellenbeständig zu sein, und einen mikrowellenbeständigen Aufbewahrungsbehälter (14).

## Claims

1. Menstrual cup (1) comprising an upper opening (2), and a receptacle (3) comprising a body (4) having a rounded longitudinal shape and a bottom (5), the cup extending longitudinally from the opening (2) to the bottom (5) and having an internal surface (6) in the concavity of the receptacle (3), and an external surface (7) in the convexity of the receptacle (3),
the body (4) comprising at least one hollow longitudinal groove (8) on the internal surface (6) of the body (4), **characterized in that** the body comprises at least one solid longitudinal groove (9) on the external surface (7) of the body (4) which coincides with said hollow longitudinal groove (8) on the internal surface (6).

2. Menstrual cup (1) according to claim 1, **characterized in that** it comprises a plurality of hollow (8) and/or solid (9) longitudinal grooves.

3. Menstrual cup (1) according to claim 2, **characterized in that** the hollow grooves (8) with which the solid grooves (9) coincide are evenly distributed at the circumference of the body (4).

4. Menstrual cup (1) according to any of the preceding claims,
**characterized in that** it comprises four hollow grooves (8) with which four solid grooves (9) correspond.

5. Menstrual cup (1) according to any of the preceding claims,
**characterized in that** it comprises at least one groove (8, 9) having a rounded or angular section.

6. Menstrual cup (1) according to any of the preceding claims,
**characterized in that** it comprises at least one groove (8, 9) which is generally flared, pear-shaped or ovoid-shaped.

7. Menstrual cup (1) according to either claim 1 or claim 2,
**characterized in that** it comprises at least one groove (8, 9) having a general serpentine shape.

8. Menstrual cup (1) according to either claim 1 or claim 2,
**characterized in that** at least one groove (8) has a tubular shape having a section corresponding to approximately one third of that of the opening.

9. Menstrual cup (1) according to any of the preceding claims,
**characterized in that** it comprises a section (9a) which is flared upward between the body (4) and the opening (2).

10. Menstrual cup (1) according to the preceding claim,
**characterized in that** the flared section (9a) has an increase in diameter of at least approximately 25% between the maximum diameter of the body and the outer diameter of the flared section (9a).

11. Kit comprising a menstrual cup (1) according to any of the preceding claims, which cup is configured to be microwave resistant, and a storage container (14) which is microwave resistant.
